# FASCICULE DE BREVET EUROPEEN

(11) **EP 3 923 841 B1**
(45) Date de publication et mention de la délivrance du brevet: **14.06.2023**
(21) Numéro de dépôt: 20704536.0
(22) Date de dépôt: 13.02.2020
(51) Int. Cl.: A61B 17/80, A61B 17/84, A61B 17/86

(54) **DISPOSITIF D'OSTÉOSYNTHÈSE À PLAQUE MUNIE D'UN ORIFICE TARAUDÉ POUR LA RÉCEPTION D'UNE VIS DE FIXATION VERROUILLÉE**
OSTEOSYNTHESEVORRICHTUNG MIT EINER MIT EINER GEWINDEBOHRUNG VERSEHENEN PLATTE ZUR AUFNAHME EINER VERRIEGELTEN BEFESTIGUNGSSCHRAUBE
OSTEOSYNTHESIS DEVICE WITH PLATE PROVIDED WITH A THREADED HOLE FOR RECEIVING A LOCKED FASTENING SCREW

(30) Priorité: 14.02.2019 FR 1901476
(43) Date de publication de la demande: 22.12.2021
(73) Titulaire: Newclip International, 2163 Luxembourg (LU)
(72) Inventeur: BALLERINI, Julien, 44100 NANTES (FR); LARCHE, Grégoire, 49300 CHOLET (FR); PODGORSKI, Jean-Pierre, 49230 SEVREMOINE (FR)
(74) Mandataire: Jacobacci Coralis Harle
(86) Numéro de dépôt international: PCT/EP2020/053791
(87) Numéro de publication internationale: WO 2020/165360

(56) Documents cités:
- DE-A1-102015 102 629
- US-A1- 2011 015 682
- US-A1- 2015 374 420
- US-A1- 2018 064 477
- US-A1- 2018 064 479

## Description

### Domaine technique de l'invention

La présente invention concerne d'une manière générale les dispositifs d'ostéosynthèse à plaque et vis qui sont utilisés par les chirurgiens pour réduire une fracture osseuse. Elle concerne plus particulièrement un dispositif d'ostéosynthèse à plaque munie d'un orifice taraudé pour la réception d'une vis de fixation verrouillée.

### Etat de la technique

Dans le domaine concerné, le document FR-2 928 259 décrit un matériel, constitué d'une plaque associée à un ensemble de vis, proposé aux chirurgiens pour réduire une fracture osseuse.

La plaque d'ostéosynthèse comporte une face inférieure destinée à venir en contact avec l'os, et une face supérieure opposée. Son épaisseur est de quelques millimètres ; et elle est traversée par une pluralité d'orifices dont certains au moins reçoivent des vis destinées à sa fixation plaquée sur le matériau osseux de réception.

Ces orifices peuvent avoir une section circulaire (section transversale à leur axe), avec une partie de pourtour en forme de tronc de cône, contre laquelle vient en contact le contour de la tête de vis associée.

Lorsque les vis de fixation ne sont pas verrouillées à la plaque, si le matériau osseux de réception n'est pas de bonne qualité, il existe un risque de retrait de ces vis par rapport à l'os et à la plaque, et de ce fait un risque de perte de réduction, ayant pour conséquence un mauvais résultat clinique.

Dans un tel cas, il est donc intéressant d'obtenir un verrouillage de la vis de fixation sur la plaque d'ostéosynthèse, ce verrouillage étant obtenu par un filetage ménagé sur le contour de la tête de vis, coopérant avec un taraudage complémentaire prévu sur le pourtour de l'orifice de réception.

Dans ce cadre, le document US-8 574 268 propose de réaliser une pluralité de dégagements (ou découpes) régulièrement répartis sur toute la hauteur du pourtour de l'orifice de plaque, de manière à définir des colonnes munies de parties de taraudage séparées les unes des autres. Une telle particularité confère au chirurgien une possibilité de réglage de l'inclinaison de l'axe de la vis par rapport à l'axe de son orifice de réception.

Cependant ce type de dispositif ne permet pas une stabilité optimale de la vis verrouillée sur la plaque, notamment dans le cas où la vis est placée dans l'axe de l'orifice associé.

### Présentation de l'invention

Afin de remédier à l'inconvénient précité de l'état de la technique, la présente invention propose un dispositif d'ostéosynthèse à plaque et vis pour fixer ladite plaque sur un os, lequel dispositif d'ostéosynthèse comprend :
- une plaque d'ostéosynthèse comprenant une face supérieure, une face inférieure destinée à venir en contact avec l'os, et un orifice de plaque s'étendant au travers de ladite plaque, depuis ladite face supérieure jusqu'à ladite face inférieure,

lequel orifice de plaque comporte une surface interne de forme générale tronconique centrée sur un axe central et convergeant depuis ladite face supérieure jusqu'à ladite face inférieure, laquelle surface interne d'orifice comprend un taraudage d'orifice, et
   - une vis comprenant une tête de vis et un corps de vis, lequel corps de vis comprend éventuellement un filetage pour la fixation de ladite vis dans l'os, et laquelle tête de vis comporte une surface périphérique munie d'un filetage destiné à coopérer avec ledit taraudage d'orifice pour le verrouillage de ladite vis sur ladite plaque d'ostéosynthèse,
ce dispositif d'ostéosynthèse étant caractérisé par le fait que ladite surface interne dudit orifice de plaque comprend :
   - un premier tronçon tronconique qui s'étend à partir de ladite face supérieure de plaque, ou à proximité de ladite face supérieure de plaque, selon un angle compris entre 30 et 60° par rapport audit axe central, lequel premier tronçon tronconique comprend une partie supérieure de premier tronçon tronconique,
   - un deuxième tronçon tronconique qui s'étend entre ledit premier tronçon tronconique et ladite face inférieure, selon un angle compris entre 5 et 15° par rapport audit axe central, lequel deuxième tronçon tronconique comprend une partie inférieure de deuxième tronçon tronconique, laquelle surface interne d'orifice comprend :
      - au moins trois dégagements supérieurs, ménagés sur une partie au moins de la hauteur dudit premier tronçon tronconique à partir de la partie supérieure de celui-ci, lesquels au moins trois dégagements supérieurs sont régulièrement espacés sur le pourtour dudit orifice de plaque selon un pas P, et
      - au moins trois dégagements inférieurs, ménagés sur une partie au moins de la hauteur dudit deuxième tronçon tronconique et qui s'étendent jusqu'à la partie inférieure de ce dernier, lesquels au moins trois dégagements inférieurs sont en nombre identique à celui desdits au moins trois dégagements supérieurs et sont décalés d'un demi pas P par rapport à ces derniers, lesquels dégagements supérieurs et dégagements inférieurs s'étendent au moins sur la profondeur dudit taraudage d'orifice et sont agencés pour autoriser un degré de liberté en angulation de ladite vis logée dans ledit orifice de plaque,
et lesquels dégagements supérieurs et dégagements inférieurs sont également agencés de sorte à préserver une partie de taraudage d'orifice sur toutes les génératrices de la surface interne d'orifice qui sont situées dans des plans parallèles audit axe central.

Une telle combinaison de caractéristiques permet une angulation de l'axe de la vis par rapport à l'axe de l'orifice de la plaque, tout en conservant une bonne stabilité du verrouillage entre la vis et la plaque. De plus, elle permet une répartition optimisée des contraintes et une bonne stabilité mécanique, en particulier lors d'un verrouillage de la vis dans l'axe de son orifice de réception.

D'autres caractéristiques non limitatives et avantageuses du dispositif d'ostéosynthèse conforme à l'invention, prises individuellement ou selon toutes les combinaisons techniquement possibles, sont les suivantes :
- au moins certains desdits dégagements supérieurs et inférieurs sont en forme de portion de sphère ;
- au moins certains desdits dégagements supérieurs et inférieurs sont en forme de portion de cylindre ;
- la surface interne de l'orifice de plaque comprend quatre dégagements supérieurs et quatre dégagements inférieurs ;
- le taraudage d'orifice est du type à double filets comprenant deux filets décalés de 180° ;
- le deuxième tronçon tronconique de la surface interne de l'orifice de plaque s'étend dans le prolongement du premier tronçon tronconique ;
- les dégagements supérieurs sont ménagés sur toute la hauteur du premier tronçon tronconique et sur une partie de la hauteur du deuxième tronçon tronconique ;
- les dégagements inférieurs sont ménagés sur toute ou pratiquement toute la hauteur dudit deuxième tronçon tronconique ;
- un lamage supérieur est prévu entre la face supérieure de la plaque d'ostéosynthèse et la partie supérieure du premier tronçon tronconique de la surface interne de l'orifice de plaque, et
- un chanfrein inférieur est prévu entre la face inférieure de la plaque d'ostéosynthèse et la partie inférieure du deuxième tronçon tronconique de la surface interne de l'orifice de plaque.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent une forme non limitative de réalisation de l'invention et où :
[Fig. 1] est une vue en perspective d'un dispositif d'ostéosynthèse selon l'invention, comprenant une plaque d'ostéosynthèse et l'une de ses vis de fixation, ladite vis de fixation étant illustrée séparée de la plaque, non engagée dans l'un des orifices de fixation ;
[Fig. 2] est une vue partielle en coupe de la plaque d'ostéosynthèse illustrée sur la figure 1, avec une vis de fixation verrouillée dans l'un des orifices circulaires de fixation ;
[Fig. 3] est une vue partielle en perspective de la plaque d'ostéosynthèse illustrée sur la figure 1, détaillant l'un de ses orifices de fixation, vu du côté de la face supérieure de la plaque ;
[Fig. 4] montre l'orifice de fixation de la figure 3, vu de dessus, c'est-à-dire vu du côté de la face supérieure de la plaque d'ostéosynthèse ;
[Fig. 5] montre l'orifice de fixation de la figure 3, vu de dessous, c'est-à-dire vu du côté de la face inférieure de la plaque d'ostéosynthèse ;
[Fig. 6] est une vue en coupe selon le plan de coupe 6-6 de la figure 5 ;
[Fig. 7] est une vue en coupe de l'ébauche de l'orifice de fixation des figures 3 à 6, avant réalisation du taraudage et des dégagements supérieurs et inférieurs ;
[Fig. 8] montre l'ébauche d'orifice de la figure 7 après réalisation de son taraudage et avant réalisation des dégagements supérieurs et inférieurs ;
[Fig. 9] montre l'ébauche d'orifice de la figure 8 après réalisation des dégagements inférieurs et avant réalisation des dégagements supérieurs.

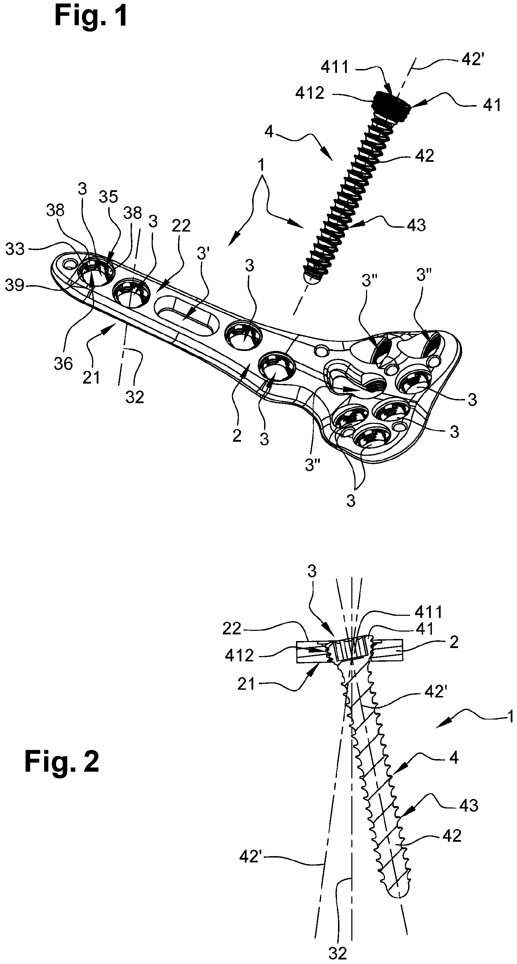

Le dispositif d'ostéosynthèse 1 illustré sur les figures 1 et 2 est adapté pour la réduction d'une fracture osseuse.

Ce dispositif d'ostéosynthèse 1 comprend une plaque d'ostéosynthèse 2, dont l'épaisseur est de quelques millimètres, délimitée par une face inférieure 21 destinée à venir en contact avec le matériau osseux de réception et par une face supérieure 22 opposée.

Cette plaque d'ostéosynthèse 2 est munie d'une pluralité d'orifices 3, 3', 3" qui traversent son épaisseur, entre sa face inférieure 21 et sa face supérieure 22, et qui sont destinés au passage de vis de fixation, pour sa fixation sur le matériau osseux de réception.

Ici, une pluralité de ces orifices 3 présentent une forme générale circulaire (plus précisément une section transversale à leur axe qui est circulaire) et leur surface interne est structurée d'une manière particulière, détaillée plus loin, pour permette un verrouillage poly-axial d'une vis de fixation 4 destinée à être implantée dans le matériau osseux de réception. L'une de ces vis de fixation 4 est représentée sur la figure 1, illustrée séparée de la plaque d'ostéosynthèse 2). La figure 2 montre cette vis de fixation 4 en position active verrouillée dans son orifice d'accueil 3 sur la plaque d'ostéosynthèse 2.

La plaque d'ostéosynthèse 2 illustrée sur la figure 1 comporte encore :
- un orifice oblong 3' qui permet le positionnement réglable en translation d'une vis de fixation, et
- trois orifices taraudés 3" chacun pour la réception et le verrouillage mono-axial d'une vis de fixation.

La vis de fixation 4 illustrée sur les figures 1 et 2 comporte - une tête 41 dont une partie au moins du contour est destinée à venir en contact avec une partie du pourtour de l'un des orifices circulaires 3, et - un corps de vis 42, d'axe longitudinal 42', muni d'un filetage 43 pour son ancrage dans le matériau osseux de réception. Une empreinte en creux 411 réalisée dans la tête de vis 41, permet les manoeuvres de rotation de la vis 4 au moyen d'un outil adapté, pour son implantation dans le matériau osseux de réception.
On notera que dans une variante de réalisation, le corps de vis 42 peut ne pas comporter de filetage.

Le contour de la tête de vis 41 a une forme générale tronconique et comporte un filetage de tête 412, ici du type à double filets composé de deux filets décalés de 180°.

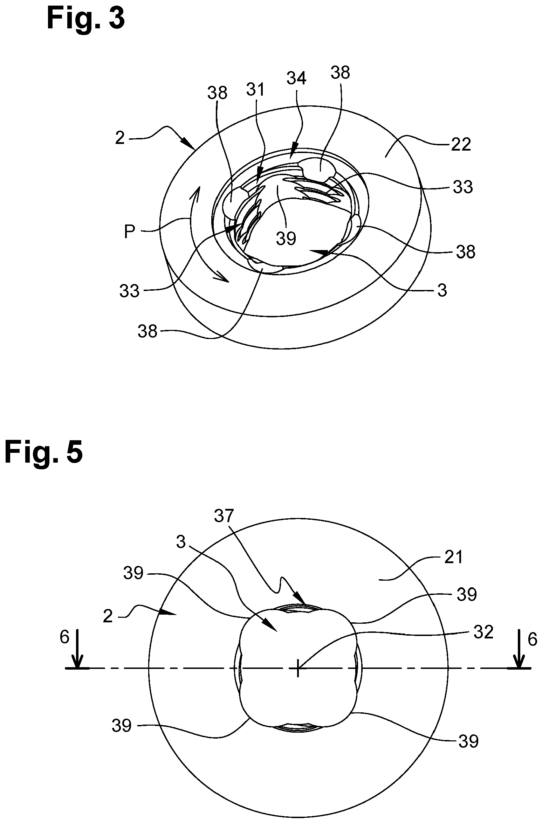

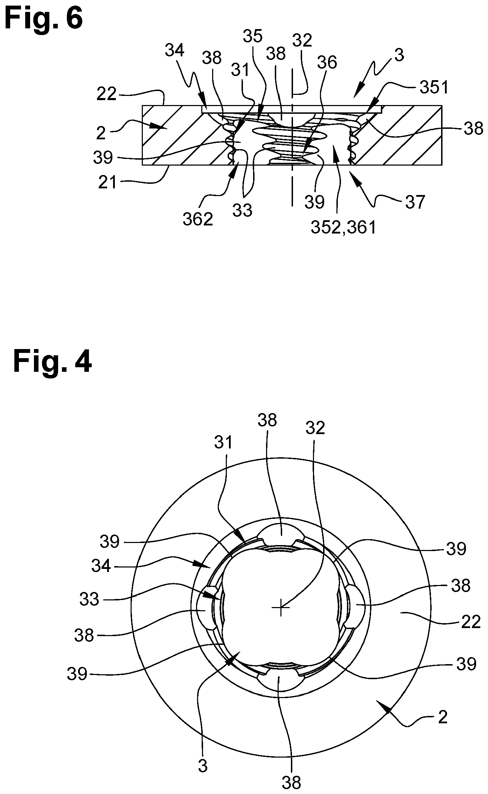

La structure de la surface interne de l'orifice 3 est détaillée sur les figures 3 à 6.

Cet orifice 3 de la plaque d'ostéosynthèse 2 comporte une surface interne 31 de forme générale tronconique centrée sur un axe central 32 et qui converge depuis la face supérieure 22 jusqu'à la face inférieure 21.

La surface interne d'orifice 31 comprend un taraudage d'orifice 33, adapté pour coopérer avec le filetage de tête 412 de la vis de fixation 4 pour permettre le verrouillage de cette vis 4 sur la plaque d'ostéosynthèse 2. ; et elle est également structurée pour permettre une angulation de la vis de fixation 4 par rapport à l'axe central 32 de l'orifice 3, de manière à conférer au chirurgien une possibilité de réglage de positionnement de cette vis de fixation 4.

Cette possibilité d'angulation est illustrée sur la figure 2 où on a représenté l'axe 42' du corps de vis 42 décalé angulairement par rapport à l'axe central d'orifice 32. Cette angulation possible peut être de l'ordre de 10° autour de l'axe central d'orifice 32 (soit 20° au global).

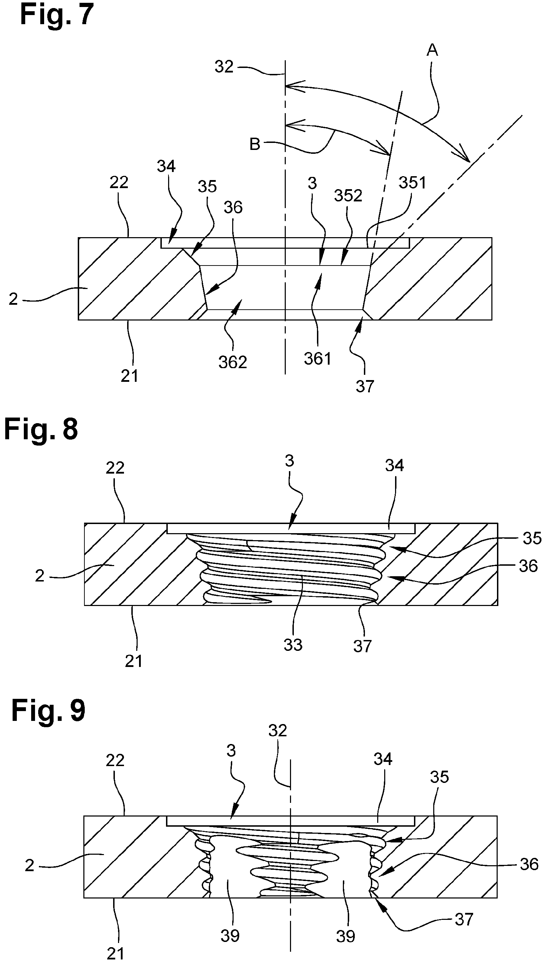

Pour cela la surface interne d'orifice 31 comprend, successivement, depuis la face supérieure 22 vers la face inférieure 21 :
- un lamage supérieur 34,
- un premier tronçon tronconique 35 qui s'étend selon un angle A (visible sur la figure 7) compris entre 30 et 60° par rapport à l'axe central 32 (de préférence, cet angle A est compris entre 40 et 50°),
- un deuxième tronçon tronconique 36 qui s'étend selon un angle B (visible sur la figure 7) compris entre 5 et 15° par rapport à l'axe central 32 (de préférence, cet angle B est compris entre 8 et 12°, et encore de préférence entre 9 et 11 °), et
- un chanfrein inférieur 37 de sortie.

Le lamage supérieur 34 s'étend entre la face supérieure 22 de la plaque d'ostéosynthèse 2 et la partie supérieure 351 du premier tronçon tronconique 35.

De préférence, le premier tronçon tronconique s'étend sur une hauteur comprise entre le quart et la moitié de la hauteur du deuxième tronçon tronconique.

Le deuxième tronçon tronconique 36 s'étend dans le prolongement du premier tronçon tronconique 35 ; ainsi, la partie inférieure 352 du premier tronçon tronconique 35 est juxtaposée avec la partie supérieure 361 du deuxième tronçon tronconique 36.

Le chanfrein inférieur 37 s'étend entre la partie inférieure 362 du deuxième tronçon tronconique 36 et la face inférieure 21 de la plaque d'ostéosynthèse 2.

D'autre part, conformément à l'invention, la surface interne d'orifice 31 comprend encore une pluralité de dégagements (ou rognures ou découpes) adaptés pour optimiser les possibilités d'inclinaison souhaitées de la vis de fixation 4, à savoir : au moins trois dégagements supérieurs 38, régulièrement répartis sur le pourtour de l'orifice 3 selon un pas P, et au moins trois dégagements inférieurs 39, également régulièrement répartis sur le pourtour de l'orifice 3 selon un pas P, ces dégagements inférieurs 39 étant en nombre identique à celui des dégagements supérieurs 38 et étant décalés d'un demi pas P par rapport à ces derniers.

Les dégagements supérieurs 38 sont avantageusement en nombre compris entre trois et huit, de préférence au nombre de quatre, comme représenté sur les figures annexées (le pas P est donc ici de 90°). Ils sont ménagés sur une partie au moins de la hauteur du premier tronçon tronconique 35 à partir de la partie supérieure 351 de celui-ci, et avantageusement sur toute la hauteur de ce premier tronçon tronconique 35 et sur une partie de la hauteur du deuxième tronçon tronconique 36.

Les dégagements inférieurs 39 sont avantageusement en nombre compris entre trois et huit, de préférence au nombre de quatre, comme représenté sur les figures annexées (le pas P est donc ici de 90°). Ils sont ménagés sur une partie au moins de la hauteur (et de préférence sur toute ou pratiquement toute la hauteur) du deuxième tronçon tronconique, jusqu'à la partie inférieure 362 de ce dernier.

Les dégagements supérieurs 38 et les dégagements inférieurs 39 s'étendent au moins sur la profondeur du taraudage d'orifice 33 et ils sont agencés pour autoriser un degré de liberté en angulation de la vis de fixation 4 logée dans l'orifice de plaque associé 3. En outre, ils sont également agencés de sorte à préserver une partie de taraudage d'orifice 33 sur toutes les génératrices de la surface interne d'orifice 31 qui sont situées dans des plans parallèles à l'axe central 32.

Une telle combinaison de caractéristiques permet d'obtenir une bonne stabilité mécanique du verrouillage entre la vis de fixation et la plaque d'ostéosynthèse (du fait de la présence d'au moins une partie de filet de taraudage sur toute coupe axiale du pourtour de l'orifice 3), tout en disposant du caractère poly-axial recherché de la vis de fixation.

Les pentes des premier et deuxième tronçons tronconiques 35, 36, ainsi que le nombre, la forme et la profondeur des dégagements supérieurs 38 et inférieurs 39, sont adaptés en fonction du compromis souhaité entre la qualité de la stabilité mécanique du verrouillage et le degré d'angularité possible.

De tels orifices 3 sont obtenus tout d'abord en réalisant une ébauche circulaire d'orifice telle qu'illustrée sur la figure 7, avec le lamage supérieur 34, le premier tronçon tronconique 35, le deuxième tronçon tronconique 36, et le chanfrein inférieur 37 de sortie.

Ensuite, comme illustré sur la figure 8, on réalise le taraudage d'orifice 33 sur toute la surface interne 31 de l'orifice 3. Ce taraudage est de préférence du type à double filets composé de deux filets décalés de 180°.
Ce taraudage 33 suit la forme de l'ébauche, c'est-à-dire qu'il suit les deux pentes de l'orifice.

Enfin on réalise les dégagements inférieurs 39 et supérieurs 38, par exemple mais non obligatoirement dans cet ordre.

La figure 9 montre l'orifice de plaque 3 en cours de préparation, après réalisation des dégagements inférieurs 39, et avant la réalisation des dégagements supérieurs 38.

Ces dégagements inférieurs 39 peuvent être en forme de portion de cylindre d'axe incliné ou non par rapport à l'axe 32 de l'orifice 3.

Les dégagements supérieurs 38 sont réalisés ensuite pour obtenir la structure finale d'orifice telle qu'illustrée sur les figures 3 à 6.

Ces dégagements supérieurs 38 peuvent être en forme de portion de sphère, ou en portion de cylindre.

## Revendications

1. Dispositif d'ostéosynthèse à plaque et vis pour fixer ladite plaque sur un os, lequel dispositif d'ostéosynthèse (1) comprend :
- une plaque d'ostéosynthèse (2) comprenant une face supérieure (22), une face inférieure (21) destinée à venir en contact avec l'os, et un orifice de plaque (3) s'étendant au travers de ladite plaque (2), depuis ladite face supérieure (22) jusqu'à ladite face inférieure (21),
lequel orifice de plaque (3) comporte une surface interne (31) de forme générale tronconique centrée sur un axe central (32) et convergeant depuis ladite face supérieure (22) jusqu'à ladite face inférieure (21),
laquelle surface interne (31) d'orifice comprend un taraudage d'orifice (33), et
- une vis (4) comprenant une tête de vis (41) et un corps de vis (42), lequel corps de vis (42) comprend éventuellement un filetage (43) pour la fixation de ladite vis (4) dans l'os, et laquelle tête de vis (41) comporte une surface périphérique munie d'un filetage (412) destiné à coopérer avec ledit taraudage d'orifice (33) pour le verrouillage de ladite vis (4) sur ladite plaque d'ostéosynthèse (2),
**caractérisé en ce que** ladite surface interne (31) dudit orifice de plaque (3) comprend :
- un premier tronçon tronconique (35) qui s'étend à partir de ladite face supérieure (22) de plaque, ou à proximité de ladite face supérieure (22) de plaque, selon un angle compris entre 30 et 60° par rapport audit axe central (32), lequel premier tronçon tronconique (35) comprend une partie supérieure de premier tronçon tronconique (351),
- un deuxième tronçon tronconique (36) qui s'étend entre ledit premier tronçon tronconique (35) et ladite face inférieure (21) de plaque, selon un angle compris entre 5 et 15° par rapport audit axe central (32), lequel deuxième tronçon tronconique (36) comprend une partie inférieure de deuxième tronçon tronconique (362),
laquelle surface interne (31) d'orifice comprend :
- au moins trois dégagements supérieurs (38), ménagés sur une partie au moins de la hauteur dudit premier tronçon tronconique (35) à partir de la partie supérieure (351) de celui-ci, lesquels au moins trois dégagements supérieurs (38) sont régulièrement espacés sur le pourtour dudit orifice de plaque (3) selon un pas P, et
- au moins trois dégagements inférieurs (39), ménagés sur une partie au moins de la hauteur dudit deuxième tronçon tronconique (36) et qui s'étendent jusqu'à la partie inférieure (362) de ce dernier,
lesquels au moins trois dégagements inférieurs (39) sont en nombre identique à celui desdits au moins trois dégagements supérieurs (38) et sont décalés d'un demi pas P par rapport à ces derniers,
lesquels dégagements supérieurs (38) et dégagements inférieurs (39) s'étendent au moins sur la profondeur dudit taraudage d'orifice (33) et sont agencés pour autoriser un degré de liberté en angulation de ladite vis (4) logée dans ledit orifice de plaque (3),
et lesquels dégagements supérieurs (38) et dégagements inférieurs (39) sont également agencés de sorte à préserver une partie de taraudage d'orifice (33) sur toutes les génératrices de la surface interne d'orifice (31) qui sont situées dans des plans parallèles audit axe central (32).

2. Dispositif d'ostéosynthèse (1) selon la revendication 1, **caractérisé en ce qu'**au moins certains desdits dégagements supérieurs (38) et inférieurs (39) sont en forme de portion de sphère.

3. Dispositif d'ostéosynthèse selon l'une quelconque des revendications 1 ou 2, **caractérisé en ce qu'**au moins certains desdits dégagements supérieurs (38) et inférieurs (39) sont en forme de portion de cylindre.

4. Dispositif d'ostéosynthèse selon l'une quelconque des revendications 1 à 3, **caractérisé en ce qu'**il comprend quatre dégagements supérieurs (38) et quatre dégagements inférieurs (39).

5. Dispositif d'ostéosynthèse selon l'une quelconque des revendications 1 à 4, **caractérisé en ce que** ledit taraudage d'orifice (33) est du type à double filets comprenant deux filets décalés de 180°.

6. Dispositif d'ostéosynthèse selon l'une quelconque des revendications 1 à 5, **caractérisé en ce que** ledit deuxième tronçon tronconique (36) s'étend dans le prolongement dudit premier tronçon tronconique (35).

7. Dispositif d'ostéosynthèse selon la revendication 6, **caractérisé en ce que** lesdits dégagements supérieurs (38) sont ménagés sur toute la hauteur dudit premier tronçon tronconique (35) et sur une partie de la hauteur dudit deuxième tronçon tronconique (36).

8. Dispositif d'ostéosynthèse selon l'une quelconque des revendications 1 à 7, **caractérisé en ce que** lesdits dégagements inférieurs (39) sont ménagés sur toute ou pratiquement toute la hauteur dudit deuxième tronçon tronconique (36).

9. Dispositif d'ostéosynthèse selon l'une quelconque des revendications 1 à 8, **caractérisé en ce qu'**il comprend :
- un lamage supérieur (34) entre la face supérieure (22) de ladite plaque d'ostéosynthèse (2) et ladite partie supérieure (351) du premier tronçon tronconique (35), et
- un chanfrein inférieur (37) entre la face inférieure (21) de ladite plaque d'ostéosynthèse (2) et ladite partie inférieure (362) du deuxième tronçon tronconique (36).

## Patentansprüche

1. Osteosynthesevorrichtung mit einer Platte und einer Schraube zum Befestigen der Platte an einem Knochen, wobei die Osteosynthesevorrichtung (1)
- eine Osteosyntheseplatte (2) mit einer Oberseite (22), einer Unterseite (21), die dazu bestimmt ist, mit dem Knochen in Kontakt zu kommen, und einem Plattenloch (3), das sich von der Oberseite (22) bis zur Unterseite (21) durch die Platte (2) erstreckt,
wobei das Plattenloch (3) eine innere Oberfläche (31) von insgesamt kegelstumpfartiger, auf eine Mittelachse (32) zentrierter und von der Oberseite (22) zur Unterseite (21) hin konvergierender Form aufweist,
wobei die innere Lochoberfläche (31) ein Lochgewinde (33) aufweist,
und
- eine Schraube (4) mit einem Schraubenkopf (41) und einem Schraubenkörper (42), wobei der Schraubenkörper (42) eventuell ein Gewinde (43) zum Befestigen der Schraube (4) im Knochen aufweist und wobei der Schraubenkopf (41) eine mit einem Gewinde (412) versehene Umfangsfläche aufweist, die dazu bestimmt ist, zum Verriegeln der Schraube (4) auf der Osteosyntheseplatte (2) mit dem Lochgewinde (33) zusammenzuwirken,
aufweist,
**dadurch gekennzeichnet, daß** die innere Oberfläche (31) des Plattenlochs (3)
- einen ersten kegelstumpfförmigen Abschnitt (35), der sich von der Plattenoberseite (22) aus oder nahe der Plattenoberseite (22) unter einem Winkel zwischen 30° und 60° gegenüber der Mittelachse (32) erstreckt, wobei der erste kegelstumpfförmige Abschnitt (35) einen oberen Teil (351) des ersten kegelstupfförmigen Abschnitts aufweist,
- einen zweiten kegelstumpfförmigen Abschnitt (36), der sich unter einem Winkel zwischen 5° und 15° gegenüber der Mittelachse (32) zwischen dem ersten kegelstumpfförmigen Abschnitt (35) und der Plattenunterseite (21) erstreckt, wobei der zweite kegelstumpfförmige Abschnitt (36) einen unteren Teil (362) des zweiten kegelstupfförmigen Abschnitts aufweist,
aufweist,
wobei die innere Lochoberfläche (31)
- mindestens drei obere Freiräume (38), die mindestens auf einem Teil der Höhe des ersten kegelstumpfförmigen Abschnitts (35) von der Oberseite (351) desselben aus eingerichtet sind, wobei die mindestens drei oberen Freiräume (38) auf dem Umfang des Plattenlochs (3) entlang eines Schritts P voneinander gleichmäßig beabstandet sind, und
- mindestens drei untere Freiräume (39), die mindestens auf einem Teil der Höhe des zweiten kegelstumpfförmigen Abschnitts (36) eingerichtet sind und sich bis zum unteren Teil (362) desselben erstrecken,
aufweist,
wobei die mindestens drei unteren Freiräume (39) zahlenmäßig gleich den mindestens drei oberen Freiräumen (38) sind und um einen halben Schritt P gegenüber den letzteren versetzt sind,
wobei sich die oberen Freiräume (38) und die unteren Freiräume (39) mindestens über die Tiefe des Lochgewindes (33) erstrecken und angeordnet sind, um einen winkelmäßigen Freiheitsgrad der in das Plattenloch (3) eingebrachten Schraube (4) zu ermöglichen,
und wobei die oberen Freiräume (38) und die unteren Freiräume (39) auch so angelegt sind, daß ein Teil des Lochgewindes (33) über alle Mantellinien der inneren Lochoberfläche (31), die in zur Mittelachse (32) parallelen Ebenen angeordnet sind, erhalten bleibt.

2. Osteosynthesevorrichtung (1) gemäß Anspruch 1, **dadurch gekennzeichnet, daß** mindestens einige der oberen (38) und der unteren (39) Freiräume die Form eines Kugelabschnitts haben.

3. Osteosynthesevorrichtung gemäß einem der Ansprüche 1 oder 2, **dadurch gekennzeichnet, daß** mindestens einige der oberen (38) und der unteren (39) Freiräume die Form eines Zylinderabschnitts haben.

4. Osteosynthesevorrichtung gemäß einem der Ansprüche 1 bis 3, **dadurch gekennzeichnet, daß** sie vier obere Freiräume (38) und vier untere Freiräume (39) aufweist.

5. Osteosynthesevorrichtung gemäß einem der Ansprüche 1 bis 4, **dadurch gekennzeichnet, daß** das Lochgewinde (33) vom Typ eines doppelten Gewindes mit zwei um 180° versetzten Gewinden ist.

6. Osteosynthesevorrichtung gemäß einem der Ansprüche 1 bis 5, **dadurch gekennzeichnet, daß** sich der zweite kegelstumpfförmige Abschnitt (36) in der Verlängerung des ersten kegelstumpfförmigen Abschnitts (35) erstreckt.

7. Osteosynthesevorrichtung gemäß Anspruch 6, **dadurch gekennzeichnet, daß** die oberen Freiräume (38) über die gesamte Höhe des ersten kegelstumpfförmigen Abschnitts (35) und über einen Teil der Höhe des zweiten kegelstumpfförmigen Abschnitts (36) eingerichtet sind.

8. Osteosynthesevorrichtung gemäß einem der Ansprüche 1 bis 7, **dadurch gekennzeichnet, daß** die unteren Freiräume (39) über die gesamte oder praktisch gesamte Höhe des zweiten kegelstumpfförmigen Abschnitts (36) eingerichtet sind.

9. Osteosynthesevorrichtung gemäß einem der Ansprüche 1 bis 8, **dadurch gekennzeichnet, daß** sie
- eine obere Senkung (34) zwischen der Oberseite (22) der Osteosyntheseplatte (2) und dem oberen Teil (351) des ersten kegelstumpfförmigen Abschnitts (35) und
- eine untere Fase (37) zwischen der Unterseite (21) der Osteosyntheseplatte (2) und dem unteren Teil (362) des zweiten kegelstumpfförmigen Abschnitts (36)
aufweist.

## Claims

1. A plate and screw osteosynthesis device for fixing said plate on a bone, which osteosynthesis device (1) comprises:
- an osteosynthesis plate (2) comprising an upper face (22), a lower face (21) intended to come into contact with the bone, and a plate hole (3) extending through said plate (2), from said upper face (22) to said lower face (21),
which plate hole (3) has an internal surface (31) of generally frustoconical shape centered on a central axis (32) and converging from said upper face (22) to said lower face (21),
which hole internal surface (31) comprises a hole internal thread (33), and
- a screw (4) comprising a screw head (41) and a screw body (42), which screw body (42) possibly comprises a thread (43) for the fixation of said screw (4) into the bone, and which screw head (41) has a peripheral surface provided with a thread (412) intended to cooperate with said hole internal thread (33) for the locking of said screw (4) to said osteosynthesis plate (2),
**characterized in that** said internal surface (31) of said plate hole (3) comprises:
- a first frustoconical section (35) that extends from said plate upper face (22), or close to said plate upper face (22), at an angle between 30 and 60° with respect to said central axis (32), which first frustoconical section (35) comprises a first frustoconical section upper portion (351),
- a second frustoconical section (36) that extends between said first frustoconical section (35) and said lower plate face (21), at an angle between 5 and 15° with respect to said central axis (32), which second frustoconical section (36) comprises a second frustoconical section lower portion (362),
which hole internal surface (31) includes:
- at least three upper undercuts (38), provided over at least part of height of said first frustoconical section (35) from the upper portion (351) of the latter, which at least three upper undercuts (38) are regularly spaced apart around the periphery of said plate hole (3) at a pitch P, and
- at least three lower undercuts (39), provided over at least part of height of said second frustoconical section (36) and that extend to the lower portion (362) of the latter,
which at least three lower undercuts (39) are in the same number as said at least three upper undercuts (38) and are offset by half the pitch P with respect to these latter,
which upper undercuts (38) and lower undercuts (39) extend at least over the depth of said hole internal thread (33) and are arranged in such a way as to allow a degree of freedom in angulation of said screw (4) housed in said plate hole (3),
and which upper undercuts (38) and lower undercuts (39) are also arranged in such a way as to preserve a portion of hole internal thread (33) on all the generatrices of the hole internal surface (31) which are located in planes parallel to said central axis (32).

2. The osteosynthesis device (1) according to claim 1, **characterized in that** at least certain of said upper (38) and lower (39) undercuts are in the form of a portion of a sphere.

3. The osteosynthesis device according to any one of claims 1 or 2, **characterized in that** at least certain of said upper (38) and lower (39) undercuts are in the form of a portion of a cylinder.

4. The osteosynthesis device according to any one of claims 1 to 3, **characterized in that** it comprises four upper undercuts (38) and four lower undercuts (39).

5. The osteosynthesis device according to any one of claims 1 to 4, **characterized in that** said hole internal thread (33) is of the double-thread type consisted of two threads offset by 180°.

6. The osteosynthesis device according to any one of claims 1 to 5, **characterized in that** said second frustoconical section (36) extends in the continuation of said first frustoconical section (35).

7. The osteosynthesis device according to claim 6, **characterized in that** said upper undercuts (38) are arranged over the whole height of said first frustoconical section (35) and over part of the height of said second frustoconical section (36).

8. The osteosynthesis device according to any one of claims 1 to 7, **characterized in that** said lower undercuts (39) are arranged over the whole or almost the whole height of said second frustoconical section (36).

9. The osteosynthesis device according to any one of claims 1 to 8, **characterized in that** it comprises:
- an upper counterbore (34) between the upper face (22) of said osteosynthesis plate (2) and said upper portion (351) of the first frustoconical section (35), and
- a lower chamfer (37) between the lower face (21) of said osteosynthesis plate (2) and said lower portion (362) of the second frustoconical section (36).
